# FASCICULE DE BREVET EUROPEEN

(11) **EP 0 930 061 B1**
(45) Date de publication et mention de la délivrance du brevet: **25.02.2004**
(21) Numéro de dépôt: 98403148.4
(22) Date de dépôt: 14.12.1998
(51) Int. Cl.: A61K 7/09

(54) **Agent oxydant à plusiers composants et procédé de déformation permanente des cheveux le mettant en oeuvre**
Oxidationsmittel von mehreren Komponenten und Verfahren für eine dauerhafte Haarverformung durch die Verwendung desselben
Oxidizing agent from a plurality of components and process for permanent hair waving using it

(30) Priorité: 30.12.1997 FR 9716718
(43) Date de publication de la demande: 21.07.1999
(73) Titulaire: L'OREAL, 75008 Paris (FR)
(72) Inventeur: Nguyen, Ly-Lan, 94240 L'Hay les Roses (FR); Sabbagh, Anne, 92500 Rueil Malmaison (FR)
(74) Mandataire: Casalonga, Axel

(56) Documents cités:
- EP-A- 0 356 665
- DE-A- 4 131 992
- GB-A- 2 116 218

## Description

La présente invention est relative à un agent oxydant à au moins deux composants comprenant une composition contenant un oxydant en solution aqueuse et une composition contenant un agent épaississant particulier, les deux compositions étant mélangées avant emploi et le mélange étant utilisé dans un procédé de traitement des cheveux, en vue d'obtenir une déformation permanente de ces derniers et au procédé mettant en oeuvre cet agent.

L'une des techniques couramment utilisée dans le domaine cosmétique pour imprimer aux cheveux une forme durable consiste à procéder à la déformation des cheveux en mettant en oeuvre un agent de réduction puis un agent oxydant.

La technique la plus usuelle pour obtenir une déformation permanente des cheveux consiste dans un premier temps à réaliser l'ouverture des liaisons disulfure (S-S) de la kératine (cystine) à l'aide d'une composition contenant un agent réducteur, puis après avoir de préférence rincé la chevelure ainsi traitée, à reconstituer dans un second temps lesdites liaisons disulfure en appliquant sur les cheveux préalablement mis sous tension, par des bigoudis ou autres, ou mis en forme ou lissés par d'autres moyens, une composition oxydante encore appelée "fixateur" de façon à donner, en définitive, la forme recherchée aux cheveux.

Cette technique permet ainsi de réaliser, soit l'ondulation des cheveux, soit leur défrisage ou leur décrêpage ou encore leur lissage.

Cette nouvelle forme, imposée aux cheveux par un traitement chimique, est durable dans le temps pendant quelques semaines et résiste notamment à l'action des lavages à l'eau ou aux shampoings et ceci par opposition aux techniques mettant en oeuvre des produits de coiffage entraînant une déformation temporaire, telle que la mise en plis, une telle déformation disparaissant au coiffage ou au shampoing.

Les compositions réductrices généralement utilisées pour la première étape d'une opération de permanente contiennent à titre d'agents réducteurs des sulfites, des bisulfites ou de préférence des thiols. Parmi ceux-ci, on peut citer plus particulièrement la cystéine et ses dérivés, la cystéamine et ses dérivés, l'acide thiolactique, l'acide thioglycolique ainsi que ses esters, notamment le thioglycolate de glycérol. L'acide thioglycolique est particulièrement efficace et constitue le produit le plus utilisé pour réduire les liaisons disulfures de la kératine.

Les agents oxydants généralement utilisés dans les compositions oxydantes sont notamment des peroxydes tels que l'eau oxygénée, les peroxyde d'urée, les bromates tels que les bromates alcalins, les persels ou un mélange de bromates alcalins et d'un persel.

Pour certaines techniques de permanentes comme par exemple lors de procédés de déformation sans bigoudis, ou lors d'un défrisage, il est préférable d'utiliser des agents oxydants suffisamment épaissis afin d'en faciliter l'application, de permettre une meilleure localisation du produit sur la chevelure, d'éviter que la composition oxydante ne coule et de permettre un maintien des cheveux dans la position désirée. De telles compositions épaissies sont divulguées dans GB 2 116 218.

Néanmoins, la formulation de compositions oxydantes épaissies est particulièrement difficile en raison de fréquents problèmes d'instabilité dans le temps. Plusieurs phénomènes se produisent généralement : on observe notamment une chute de la viscosité du produit et/ou une diminution du titre en agent oxydant.

Si on sépare le système épaississant de l'oxydant (système multicompartiments) on se heurte à la difficulté d'obtenir un épaississement homogène conséquent suffisamment rapidement et facilement après mélange.

Les demandes EP 0 356 665 et DE 41 31 992 divulguent des compositions fixatrices oxydantes pour permanentes, éventuellement sous forme de deux composants conservés séparément et mélangés immédiatement avant emploi. Les compositions oxydantes divulguées peuvent être épaissies, mais ces documents ne fournissent aucune information concernant la nature chimique d'éventuels agents épaississants.

La présente invention permet de résoudre ces différents problèmes en mettant à disposition un agent oxydant à deux composants comprenant un premier composant constitué par une composition contenant un oxydant en solution aqueuse et un second composant constitué par une composition contenant un polymère épaississant sous une forme particulière.

Les compositions de la présente invention présentent notamment l'avantage d'être très facilement et rapidement réalisables, permettant ainsi d'obtenir une composition oxydante épaissie et homogène pratiquement immédiatement, par simple mélange des deux composants.

De plus, les compositions oxydantes de la présente invention présentent des textures particulièrement adaptées à toutes les applications et notamment à celle sur cheveux non enroulés sur bigoudis; la composition étant facilement applicable, elle ne coule pas et permet le maintient des cheveux dans la position désirée.

Un objet de l'invention est constitué par un agent oxydant à au moins deux composants comprenant une composition contenant un oxydant en solution aqueuse et une composition contenant un polymère épaississant.

Un autre objet de l'invention est constitué par un procédé de déformation permanente des cheveux mettant en oeuvre l'application d'une composition réductrice suivie par l'application d'une composition obtenue par mélange avant emploi des deux composants de l'agent oxydant susmentionné.

D'autres objets de l'invention apparaîtront à la lecture de la description et des exemples qui suivent.

L'agent oxydant conforme à l'invention est essentiellement caractérisé par le fait qu'il comprend :
(1) un premier composant (A) constitué par une composition contenant en milieu aqueux au moins un oxydant;
(2) un second composant (B) constitué par une composition contenant en milieu aqueux au moins un polymère épaississant en dispersion aqueuse ou huileuse ou en émulsion inverse;
les composants (A) et (B) étant destinés à être mélangés l'un avec l'autre au moment de l'emploi en vue d'obtenir une composition oxydante prête à l'emploi destinée à être appliquée sur les cheveux en vue de reconstituer les liaisons disulfures de ceux-ci.

Les oxydants du composant (A) peuvent notamment être choisis parmi le peroxyde d'hydrogène ou eau oxygénée, le peroxyde d'urée, les bromates de métaux alcalins, les persels tels que les perborates et persulfates. Le système oxydant peut contenir des enzymes comme les peroxydases, ou être constitués par une enzyme telle qu'une oxydo réductases à 2 électrons associée à des donneurs. L'utilisation du peroxyde d'hydrogène ou des enzymes est particulièrement préférée.

La concentration en eau oxygénée de la composition oxydante prête à l'emploi peut varier de 1 à 10 volumes, mais de préférence est de l'ordre de 8 volumes.

L'eau oxygénée peut être stabilisée, par exemple par la phénacétine, l'acétaniline, les phosphates mono- et trisodiques ou par les sulfates d'hydroxy-8 quinoléïne.

La concentration en bromates alcalins est de 1 à 12% et celle en persels de 0,1 à 15% en poids par rapport au poids total de la composition oxydante prête à l'emploi.

Les polymères épaississants du composant (B) sont choisis de préférence
- les copolymères d'acrylate d'ammonium/acrylamide, en émulsion inverse E/H tel que le BOZEPOL C commercialisé par HOECHST;
- les copolymères d'acrylamide/acrylamido-2-méthyl propane sulfonique en émulsion inverse tel que le SEPIGEL 305 commercialisé par SEPPIC;
- les copolymères d'acrylate de sodium/acrylamide en émulsion inverse tel que le SEPIGEL 901 commercialisé par SEPPIC
- les copolymères de chlorure de méthacrylate de triméthyl éthyl ammonium/acrylate, en dispersion huileuse tel que le SALCARE SC 92, commercialisé par ALLIED COLLOIDS
- les homopolymères de chlorure de méthacrylate d'éthyle triméthyl ammonium, réticulé en dispersion huileuse tel que le SALCARE SC 95 commercialisé par ALLIED COLLOIDS.
- les hydroxypropyl méthylcellulose, en dispersion aqueuse tel que l'AGU D 3295A commercialisé par HERCULES.

Le polymère épaississant est présent dans la composition (B) dans des proportions telles que la composition prête à l'emploi résultant du mélange des composants (A) et (B) présente une viscosité suffisante pour ne pas couler sur le cuir chevelu et/ou pour maintenir les déformations imprimées aux cheveux.

Les polymères épaississants sont utilisés de préférence dans des proportions comprises entre 0,1 et 30% en poids par rapport au poids total de la composition prête à l'emploi.

Le pH du composant (A) et celui du composant (B) peuvent être ajustés de façon à obtenir un pH de la composition prête à l'emploi compris entre 2 et 9 et de préférence entre 2,5 et 7,5.

L'agent oxydant peut également contenir, soit dans son composant (A), soit dans son composant (B), soit dans le mélange prêt à l'emploi, des agents tensio-actifs, des agents traitants de nature anionique, non-ionique ou amphotère.

Les agents tensio-actifs utilisés sont ceux couramment utilisés dans les compositions oxydantes de permanentes et peuvent être de type non-ionique, anionique, cationique ou amphotère. On peut notamment citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que des tensio-actifs non-ioniques de la famille des hydroxypropyléthers.

Ces agents tensio-actifs sont généralement utilisés dans des proportions telles que dans la composition résultant du mélange des composants (A) et (B), leur proportion maximale soit de l'ordre de 30% en poids, et de préférence comprise entre 0,5 et 10% en poids par rapport au poids total de la composition.

On peut utiliser comme agents traitants des silicones volatiles ou non, linéaires ou cycliques ou leurs mélanges, les polydiméthylsiloxanes, les polyorganosiloxanes quaternisés, ceux décrits dans la demande de brevet français 2 535 730, les polyorganosiloxanes à groupement amino-alkyle modifiés par des groupements alcoxycarbonylakyle tels que décrits dans le brevet US-A-4 749 732, les polyorganosiloxanes tels que les copolymères de polydiméthylsiloxane-polyoxyalkyle tel que le diméthicone copolyol, un polydiméthylsiloxane à groupements terminaux stéaroxy-(stéaroxydiméthicone), un copolymère polydiméthylsiloxane dialkylammonium acétate ou un copolymère polydiméthylsiloxane polyalkylétaîne décrit dans GB-A-2 197 352, des polysiloxanes organomodifiés par des groupements mercapto ou mercapto-alkyle tels que décrits dans FR-B-1 530 369 et EP-A-0 295 780, ainsi que des silanes tels que le stéroxy-triméthylsilane.

On peut également utiliser d'autres ingrédients traitants tels que des cires, des polymères choisis parmi les polymères. cosmétiquement acceptables qui peuvent être des polymères cationiques, anioniques, non-ioniques ou amphotères, des agents de gonflement et de pénétration permettant de renforcer l'efficacité du réducteur tels que le diméthylisosorbitol, l'urée et ses dérivés, la pyrrolidone, les n-alkylpyrrolidone, la thiamorpholinone, les alkyléthers d'alkylèneglycol ou de dialkylèneglycol tels que par exemple le monométhyléthyléther de propylèneglycol, le monométhyléther de dipropylèneglycol, des alcanediols en C₃-C₆ tels que par exemple le propanediol-1,2, l'imidazolidinone-2, ainsi que d'autres composés tels que des alcools gras, des dérivés de lanoline, des céramides et notamment des céramides elles-mêmes, les glycocéramides, les pseudocéramides décrits notamment dans FR-A-95/1399, et dans DOWNING Journal of Lipid Research, Vol. 35, p. 2060, 1994, ou dans FR-A-2 673 197, EP-A-0227994, WO-94/07844, WO-92/05674, des ingrédients actifs tels que l'acide pantothénique et le panthenol, des agents anti-chute, des agents anti-pelliculaires, des agents de mise en suspension, des agents séquestrants, des agents opacifiants, des colorants, des filtres solaires, ainsi que des parfums et des conservateurs.

Le procédé conforme à l'invention est essentiellement caractérisé par le fait :
- qu'on applique une composition contenant au moins un agent réducteur sur la chevelure mouillée ou non ceci avant, pendant ou après l'étape de mise sous tension des cheveux par un moyen mécanique ou mis en forme par tout moyen manuel
- qu'on procède à un mélange d'une composition contenant en milieu aqueux un oxydant telle que définie ci-dessus avec une composition contenant un polymère épaississant en dispersion aqueuse ou huileuse ou en émulsion inverse telle que définie ci-dessus;
- qu'on applique après un temps de pose suffisant pour permettre la réduction des liaisons disulfures des cheveux et la mise en forme des cheveux et après un éventuel rinçage, la composition prête à l'emploi ainsi obtenue sur la chevelure;
- qu'on procède après un temps de pose suffisant au rinçage final.

Cette composition réductrice contient au moins un agent réducteur préférentiellement choisi parmi l'acide thioglycolique, l'acide thiolactique, la cystéine, la cystéamine, le thioglycérol, le thioglycolate de glycérol ou l'un de leurs sels cosmétiquement acceptables tels que plus particulièrement, les chlorhydrates, les bromhydrates, les citrates, les acétates et les sulfates.

Ce réducteur est utilisé dans des proportions suffisantes pour réduire les liaisons disulfures et de préférence, dans des proportions comprises entre 1 et 25% et en particulier dans des proportions comprises entre 3 et 25% en poids par rapport au poids total de la composition réductrice.

Les compositions réductrices peuvent en outre également contenir des agents alcalins, des agents tensioactifs ou des agents traitants tels que définis ci-dessus.

Cette application peut être réalisée, avant, pendant ou après l'habituelle étape de mise sous tension des cheveux sous une forme correspondant à la forme finale désirée pour ces derniers, telle que des boucles.

Cette étape peut être mise en oeuvre par tous moyens mécaniques appropriés et connus tels que par exemple des rouleaux, bigoudis, etc...

Il est aussi possible de mettre en oeuvre le procédé sans utiliser de matériel de mise sous tension des cheveux, c'est-à-dire en appliquant simplement la composition réductrice à l'aide des doigts ou du peigne ce qui permet de sculpter la chevelure afin de maintenir les cheveux dans une position désirée telle que des boucles, des crans ou des épis.

Selon une étape facultative du procédé de l'invention, on peut, après application de la composition réductrice, soumettre la chevelure à un traitement thermique par chauffage à température comprise entre 30 et 60° C. Ce chauffage permet éventuellement d'ajuster le degré final de frisure du cheveux.

Dans la pratique, cette opération peut être conduite au moyen d'un casque de coiffure, d'un sèche cheveux, d'un dispensateur de rayons infrarouges et d'autres appareils chauffants classiques.

Il est également possible, bien entendu, de travailler à température ambiante.

On peut procéder ensuite au rinçage des cheveux, ce qui est réalisé principalement lorsque les cheveux ont une mise en forme par mise sous tension notamment par des rouleaux, bigoudis ou autres.

Il est possible de ne pas procéder à l'étape de rinçage en particulier lorsque les cheveux ont été mis en forme par des moyens autres que des moyens mécaniques.

De façon générale, avant de procéder au rinçage ou à l'application de la composition oxydante, on laisse reposer pendant quelques minutes, généralement entre 2 et 30 minutes, et de préférence entre 5 et 20 minutes, la chevelure sur laquelle a été appliquée la composition réductrice, ceci de façon à bien laisser le temps au réducteur d'agir correctement sur les cheveux.

Pendant cette phase d'attente, on prend soin que les cheveux ne sèchent pas complètement et restent ainsi humides jusqu'au moment de la mise en oeuvre de l'étape suivante.

On peut ainsi utiliser dans ce but des bonnets ou des gels de protection.

Avant l'emploi, le composant (B) défini ci-dessus est mélangé avec le composant (A) défini ci-dessus.

Préférentiellement, on mélange 1 à 99% en particulier 60 à 98,5% en poids par rapport au poids total de la composition prête à l'emploi de composant (A) contenant un oxydant avec 99 à 1% en particulier 40 à 1,5% en poids par rapport au poids total de la composition prête à l'emploi de composant (B) contenant un polymère épaississant en dispersion aqueuse ou huileuse ou en émulsion inverse.

Après un rinçage éventuel, on applique, et cela après l'étape de mise en forme des cheveux, la composition prête à l'emploi obtenue par le mélange du composant (A) au composant (B).

Dans le cas où une mise sous tension par un moyen mécanique a été effectuée, on peut retirer de la chevelure les moyens mécaniques ou les bigoudis et analogues qui maintenaient les cheveux sous tension selon la forme désirée tout au long du traitement, avant ou après l'étape de fixation.

En tout état de cause, on procède après un temps de pose de 5 à 30 minutes, en particulier de 5 à 15 minutes, à un rinçage abondant à l'eau des cheveux ainsi traités.

Les exemples suivants sont destinés à illustrer l'invention sans présenter un caractère limitatif:

### EXEMPLE 1

### 1) Partie A

| | |
|---|---|
| SEPIGEL 305 | 8 g |

(copolymère acrylamide/ acrylamido 2-méthyl propane sulfonique sel de sodium en émulsion inverse à 40% dans eau/isoparaffine)

### 2) Partie B

- Eau oxygénée à 50% 4,8 g
- Stabilisants 0,2 g
- Parfum 0,5 g
- Alcool oléique oxyéthyléné(20 OE) 1 g
- acide citrique qs PH 3
- Eau déminéralisée qs 92 g

Au moment de l'emploi, on introduit dans un shaker la partie A et la partie B. On secoue et on obtient immédiatement un gel crème blanc homogène, prêt pour l'application.

### EXEMPLE 2

### Partie A

| | |
|---|---|
| BOZEPOL C(HOECHST) | 6 g |

(copolymère acrylate d'ammonium / acrylamide(95/5) en émulsion inverse E/H)

### Partie B

- Eau oxygénée à 50% 4,8 g
- Stabilisants 0,2 g
- Parfum 0,5 g
- Alcool oléique oxyéthyléné (20 OE) 1g
- acide citrique qs pH 3
- Eau déminéralisée sqp 94 g

Au moment de l'emploi, on introduit dans un shaker la partie A et la partie B. On secoue et on obtient immédiatement un gel crème blanc homogène, prêt pour l'application.

## Revendications

1. Agent oxydant destiné à être utilisé dans un procédé de déformation de permanente des cheveux, **caractérisé par le fait qu'**il comprend au moins :
- un premier composant constitué par une composition contenant en milieu aqueux au moins un oxydant,
- un second composant constitué par une composition contenant en milieu aqueux au moins un polymère épaississant choisi parmi les copolymères d'acrylate d'ammonium/acrylamide, les copolymères acrylamide/acrylamido 2-méthyl propane sulfonique. les copolymères d'acrylate de sodium/ acrylamide, les copolymères de chlorure de méthacrylate de triméthyl éthyl ammonium/acrylate. les homopolymères de chlorure de méthacrylate d'éthyl triméthyl ammonium réticulés et les hydroxypropylméthylcelluloses, en dispersion aqueuse ou huileuse ou en émulsion inverse;
le premier et le second composant étant destinés à être mélangés l'un avec l'autre au moment de l'emploi en vue d'obtenir une composition oxydante prête à l'emploi destinée à être appliquée sur des cheveux en vue de reconstituer les liaisons disulfures de ceux-ci.

2. Agent selon la revendication 1, **caractérisé par le fait que** l'oxydant est choisi parmi le peroxyde d'hydrogène, le peroxyde d'urée, les bromates de métaux alcalins, les persels tels que les perborates et persulfates.

3. Agent selon la revendication 1 , **caractérisé par le fait qu'**il contient une enzyme telle qu'une peroxydase ou une oxydoréductase à 2 électrons.

4. Agent selon l'une quelconque des revendications 1 à 3, **caractérisé par le fait que** le polymère épaississant est présent dans des proportions après mélange comprises entre 0,1 et 30% en poids par rapport au poids total de la composition prête à l'emploi.

5. Agent selon l'une quelconque des revendications 1 à 4, **caractérisé par le fait que** la composition contenant en milieu aqueux au moins un oxydant et/ou la composition contenant au moins un polymère épaississant en dispersion ou en émulsion inverse et/ou la composition prête à l'emploi contient en outre, des agents tensio-actifs anioniques, non-ioniques, cationiques ou amphotères.

6. Agent selon l'une quelconque des revendications 1 à 5. **caractérisé par le fait que** la composition contenant en milieu aqueux au moins un oxydant et/ou la composition contenant au moins un polymère épaississant en dispersion ou en émulsion inverse et/ou la composition prête à l'emploi contient en outre, des agents traitants choisis parmi les silicones, des cires, des polymères, des agents de gonflement et de pénétration, des alcools gras, des dérivés de lanoline, des céramides, des ingrédients actifs, des agents anti-chute, anti-pelliculaires, des agents de mise en suspension, des agents séquestrants, des agents opacifiants, des colorants. des filtres solaires siliconés ou non, des conservateurs, des parfums.

7. Procédé de déformation permanente des cheveux, **caractérisé par le fait :**
- **qu'**on applique une composition contenant au moins un agent réducteur sur la chevelure avant, pendant ou après l'étape de mise sous tension des cheveux par un moyen mécanique ou mis en forme par tous moyens manuels
- **qu'**on procède à un mélange d'une composition contenant en milieu aqueux un oxydant telle que définie dans l'une quelconque des revendications 1 à 6 avec une composition contenant un polymère épaississant en dispersion aqueuse ou huileuse ou en émulsion inverse telle que définie dans l'une quelconque des revendications 1 à 6;
- **qu'**on applique après un temps de pose suffisant pour permettre la réduction des liaisons disulfures des cheveux et après l'étape de mise en forme des cheveux la composition prête à l'emploi ainsi obtenue sur la chevelure;
- **qu'**on procède après un temps de pose suffisant pour permettre la déformation permanente, au rinçage.

8. Procédé selon la revendication 7. **caractérisé par le fait que** l'on mélange 1 à 99% en poids par rapport au poids total de la composition prête à l'emploi de la composition contenant en milieu aqueux un oxydant avec 99 à 1% en poids par rapport au poids total de la composition prête à l'emploi de composition contenant un polymère épaississant en dispersion aqueuse ou huileuse ou en émulsion inverse.

9. Procédé selon la revendication 7 ou 8, **caractérisé par le fait que** le pH de la composition prête à l'emploi est compris entre 2 et 9.

10. Procédé selon la revendication 9, **caractérisé par le fait que** le pH de la composition prête à l'emploi est compris entre 2,5 et 7.

11. Procédé selon l'une quelconque des revendications 7 à 10 **caractérisé par le fait que** la composition oxydante est maintenue au contact des cheveux pendant 5 à 30 mn.

12. Procédé selon la revendication 11, **caractérisé par le fait que** les cheveux sont rincés avant l'application de la composition oxydante.

13. Procédé selon l'une quelconque des revendications 7 à 12, **caractérisé par le fait que** la composition réductrice contient un agent réducteur choisi parmi l'acide thioglycolique, l'acide thiolactique, la cystéine, la cystéamine, le thioglycérol et le thioglycolate de glycérol ou l'un de leurs sels cosmétiquement acceptables tels que plus particulièrement les chlorhydrates, les bromhydrates, les citrates, les acétates et les sulfates.

14. Procédé selon l'une quelconque des revendications 7 à 13. **caractérisé par le fait que** l'on maintient la composition oxydante au contact des cheveux et que l'on rince ensuite à l'eau.

## Claims

1. Oxidizing agent intended to be used in a process for permanently shaping the hair, **characterized in that** it comprises at least:
- a first component consisting of a composition containing at least one oxidizing agent in aqueous medium,
- a second component consisting of a composition containing at least one thickening polymer chosen from ammonium acrylate/ acrylamide copolymers, acrylamide/2-acrylamidomethylpropanesulphonic acid copolymers, sodium acrylate/acrylamide copolymers, copolymers of trimethylethylammonium methacrylate chloride/acrylate, crosslinked homopolymers of ethyltrimethylammonium methacrylate chloride and hydroxypropylmethylcelluloses in aqueous medium, as an aqueous or oily dispersion or as a reverse emulsion;
the first and the second component being intended to be mixed with each other at the time of use in order to obtain a ready-to-use oxidizing composition intended to be applied to the hair in order to reform the hair's disulphide linkages.

2. Agent according to Claim 1, **characterized in that** the oxidizing agent is chosen from hydrogen peroxide, urea peroxide, alkali metal bromates and persalts such as perborates and persulphates.

3. Agent according to Claim 1, **characterized in that** it contains an enzyme such as a peroxidase or a 2-electron oxidoreductase.

4. Agent according to any one of Claims 1 to 3, **characterized in that** the thickening polymer is present in proportions, after mixing, of between 0.1 and 30% by weight relative to the total weight of the ready-to-use composition.

5. Agent according to any one of Claims 1 to 4, **characterized in that** the composition containing at least one oxidizing agent in aqueous medium and/or the composition containing at least one thickening polymer as a dispersion or as a reverse emulsion and/or the ready-to-use composition also contains anionic, nonionic, cationic or amphoteric surfactants.

6. Agent according to any one of Claims 1 to 5, **characterized in that** the composition containing at least one oxidizing agent in aqueous medium and/or the composition containing at least one thickening polymer as a dispersion or as a reverse emulsion also contains treating agents chosen from silicones, waxes, polymers, swelling agents, penetrating agents, fatty alcohols, lanolin derivatives, ceramides, active ingredients, agents for preventing hair loss, antidandruff agents, suspending agents, sequestering agents, opacifiers, dyes, silicone or non-silicone sunscreens, preserving agents and fragrances.

7. Process for permanently reshaping the hair, **characterized in that**:
- a composition containing at least one reducing agent is applied to the moistened or dry hair, before, during or after the step of placing the hair under tension by a mechanical means or shaping by any manual means;
- a composition containing an oxidizing agent as defined in any one of Claims 1 to 6 in aqueous medium is mixed with a composition containing a thickening polymer, as an aqueous or oily dispersion or as a reverse emulsion as defined in any one of Claims 1 to 6;
- after a period of time left on the hair which is sufficient to allow the reduction of the disulphide linkages of the hair and after the step of shaping the hair, the ready-to-use composition thus obtained is applied to the hair;
- after a period of time left on the hair which is sufficient to allow permanent reshaping, rinsing is carried out.

8. Process according to Claim 7, **characterized in that** 1 to 99% by weight, relative to the total weight of the ready-to-use composition, of the composition containing an oxidizing agent in aqueous medium is mixed with 99 to 1%, relative to the total weight of the ready-to-use composition, of the composition containing a thickening polymer as an aqueous or oily dispersion or as a reverse emulsion.

9. Process according to Claim 7 or 8, **characterized in that** the pH of the ready-to-use composition is between 2 and 9.

10. Process according to Claim 9, **characterized in that** the pH of the ready-to-use composition is between 2.5 and 7.

11. Process according to any one of Claims 7 to 10, **characterized in that** the oxidizing composition is kept in contact with the hair for 5 to 30 min.

12. Process according to Claim 11, **characterized in that** the hair is rinsed before applying the oxidizing composition.

13. Process according to any one of Claims 7 to 12, **characterized in that** the reducing composition contains a reducing agent chosen from thioglycolic acid, thiolactic acid, cysteine, cysteamine, thioglycerol and glyceryl thioglycolate or one of the cosmetically acceptable salts thereof, such as, more particularly, the hydrochlorides, hydrobromides, citrates, acetates and sulphates.

14. Process according to any one of Claims 7 to 13, **characterized in that** the oxidizing composition is kept in contact with the hair and the hair is then rinsed with water.

## Patentansprüche

1. Oxidationsmittel, das für die Verwendung in einem Verfahren zur permanenten Verformung der Haare vorgesehen ist, **dadurch gekennzeichnet, dass** es mindestens umfasst:
- eine erste Komponente, die aus einer Zusammensetzung besteht, die in wässrigem Medium mindestens eine oxidierende Verbindung enthält;
- eine zweite Komponente, die aus einer Zusammensetzung besteht, die in wässrigem Medium mindestens ein verdickendes Polymer enthält, das ausgewählt ist unter den Ammoniumacrylat/Acrylamid-Copolymeren, den Acrylamid/Acrylamido-2-methylpropansulfonsäure-Copolymeren, den Natriumacrylat/Acrylamid-Copolymeren, den Trimethylammonioethylmethacrylatchlorid/Acrylat-Copolymeren, den vernetzten Homopolymeren aus Trimethylammonioethylmethacrylatchlorid und den Hydroxypropylmethylcellulosen, in wässriger oder öliger Dispersion oder in inverser Emulsion;
wobei die erste und die zweite Komponente dafür vorgesehen sind, zum Zeitpunkt der Anwendung miteinander vermischt zu werden, um eine gebrauchsfertige oxidierende Zusammensetzung zu erhalten, die dafür vorgesehen ist, auf die Haare aufgetragen zu werden, um die Disulfid-Bindungen der Haare wieder auszubilden.

2. Mittel nach Anspruch 1, **dadurch gekennzeichnet, dass** die oxidierende Verbindung unter Wasserstoffperoxid, Harnstoffperoxid, Alkalimetallbromaten, Persalzen, wie den Perboraten und den Persulfaten, ausgewählt ist.

3. Mittel nach Anspruch 1, **dadurch gekennzeichnet, dass** es ein Enzym enthält, wie eine Peroxidase oder eine Oxidoreduktase, die 2 Elektronen überträgt.

4. Mittel nach einem der Ansprüche 1 bis 3, **dadurch gekennzeichnet, dass** das verdickende Polymer nach dem Mischen in einem Mengenanteil enthalten ist, der im Bereich von 0,1 bis 30 Gew.-%, bezogen auf das Gesamtgewicht der gebrauchsfertigen Zusammensetzung, liegt.

5. Mittel nach einem der Ansprüche 1 bis 4, **dadurch gekennzeichnet, dass** die Zusammensetzung, die in wässrigem Medium mindestens eine oxidierende Verbindung enthält, und/oder die Zusammensetzung, die mindestens ein verdickendes Polymer in Dispersion oder in inverser Emulsion enthält, und/oder die gebrauchsfertige Zusammensetzung außerdem anionische, nicht-ionische, kationische oder amphotere grenzflächenaktive Stoffe enthält.

6. Mittel nach einem der Ansprüche 1 bis 5, **dadurch gekennzeichnet, dass** die Zusammensetzung, die in wässrigem Medium mindestens eine oxidierende Verbindung enthält, und/oder die Zusammensetzung, die mindestens ein verdickendes Polymer in Dispersion oder in inverser Emulsion enthält, und/oder die gebrauchsfertige Zusammensetzung außerdem Behandlungsmittel enthält, die unter Siliconen, Wachsen, Polymeren, Quellmitteln und Penetrationshilfsmitteln, Fettalkoholen, Lanolinderivaten, Ceramiden, Wirkstoffen, Wirkstoffen gegen Haarausfall, Antischuppenmitteln, Suspendierhilfen, Maskierungsmitteln, Trübungsmitteln, Farbmitteln, Sonnenschutzfiltem, die gegebenenfalls Siliconfilter sind oder siliconhaltig sind, Konservierungsmitteln, Parfüms ausgewählt sind.

7. Verfahren zur permanenten Verformung der Haare, **dadurch gekennzeichnet, dass**
- eine Zusammensetzung, die mindestens eine reduzierende Verbindung enthält, vor, während oder nach dem Schritt, in dem die Haare mit einem mechanischen Mittel unter Spannung gesetzt werden oder mit einem beliebigen manuellen Mittel in eine Form gebracht werden, auf das Haar aufgetragen wird;
- eine Zusammensetzung, die in wässrigem Medium eine oxidierende Verbindung enthält, die wie in einem der Ansprüche 1 bis 6 definiert ist, mit einer Zusammensetzung, die ein verdickendes Polymer in wässriger oder öliger Dispersion oder in inverser Emulsion enthält, die wie in einem der Ansprüche 1 bis 6 definiert ist, vermischt wird;
- nach einer Einwirkungszeit, die ausreichend ist, um die Reduktion der Disulfid-Bindungen der Haare zu ermöglichen, und nach dem Schritt, in dem die Haare in Form gebracht wurden, die so erhaltene gebrauchsfertige Zusammensetzung auf die Haare aufgetragen wird;
- nach einer Einwirkungszeit, die ausreichend ist, um die permanente Verformung zu ermöglichen, die Haare gespült werden.

8. Verfahren nach Anspruch 7, **dadurch gekennzeichnet, dass** 1 bis 99 Gew.-%, bezogen auf das Gesamtgewicht der gebrauchsfertigen Zusammensetzung, der Zusammensetzung, die in wässrigem Medium eine oxidierende Verbindung enthält, mit 99 bis 1 Gew.-%, bezogen auf das Gesamtgewicht der gebrauchsfertigen Zusammensetzung, der Zusammensetzung, die ein verdickendes Polymer in wässriger oder öliger Dispersion oder in inverser Emulsion enthält, vermischt werden.

9. Verfahren nach Anspruch 7 oder 8, **dadurch gekennzeichnet, dass** der pH-Wert der gebrauchsfertigen Zusammensetzung im Bereich von 2 bis 9 liegt.

10. Verfahren nach Anspruch 9, **dadurch gekennzeichnet, dass** der pH-Wert der gebrauchsfertigen Zusammensetzung im Bereich von 2,5 bis 7 liegt.

11. Verfahren nach einem der Ansprüche 7 bis 10, **dadurch gekennzeichnet, dass** die oxidierende Zusammensetzung während eines Zeitraums von 5 bis 30 min mit den Haaren in Kontakt gehalten wird.

12. Verfahren nach Anspruch 11, **dadurch gekennzeichnet, dass** die Haare gespült werden, bevor die oxidierende Zusammensetzung aufgetragen wird.

13. Verfahren nach einem der Ansprüche 7 bis 12, **dadurch gekennzeichnet, dass** die reduzierende Zusammensetzung ein Reduktionsmittel enthält, das unter Thioglykolsäure, Thiomilchsäure, Cystein, Cysteamin, Thioglycerin und Thioglycolsäureglycerinester und deren kosmetisch akzeptablen Salzen, wie vor allem den Hydrochloriden, den Hydrobromiden, den Citraten, den Acetaten und den Sulfaten, ausgewählt wird.

14. Verfahren nach einem der Ansprüche 7 bis 13, **dadurch gekennzeichnet, dass** die oxidierende Zusammensetzung im Kontakt mit den Haaren gehalten wird und dass die Haare anschließend gespült werden.
